# EUROPEAN PATENT SPECIFICATION

(11) **EP 2 448 967 B1**
(45) Date of publication and mention of the grant of the patent: **15.04.2015**
(21) Application number: 10794608.9
(22) Date of filing: 28.06.2010
(51) Int. Cl.: A01K 67/027, C07K 16/00, A61K 39/395

(54) **NON-HUMAN MAMMAL MODEL OF HUMAN HEMATOPOIETIC CANCER**
NICHTMENSCHLICHES SÄUGETIERMODELL FÜR MENSCHLICHE HÄMATOPOETISCHE TUMORE
MODÈLE MAMMIFÈRE NON HUMAIN D'UN CANCER HÉMATOPOÏÉTIQUE HUMAIN

(30) Priority: 29.06.2009 US 221438 P
(43) Date of publication of application: 09.05.2012
(73) Proprietor: Leskov, Ilya B., Cambridge, MA 02138 (US); Drake, Adam C., Somerville, MA 02143 (US); Khoury, Maroun, Somerville, MA 02143 (US); Chen, Jianzhu, Lexington, MA 02420 (US); Pallasch, Christian, Cambridge, MA 02141 (US); Hemann, Michael, Cambridge, MA 02139 (US)
(72) Inventor: Leskov, Ilya B., Cambridge, MA 02138 (US); Drake, Adam C., Somerville, MA 02143 (US); Khoury, Maroun, Somerville, MA 02143 (US); Chen, Jianzhu, Lexington, MA 02420 (US); Pallasch, Christian, Cambridge, MA 02141 (US); Hemann, Michael, Cambridge, MA 02139 (US)
(74) Representative: Lock, Graham James
(86) International application number: PCT/US2010/040221
(87) International publication number: WO 2011/002721

(56) References cited:
- US-A1- 2006 063 732
- US-A1- 2006 160 129
- US-A1- 2007 118 914
- US-A1- 2008 118 477
- US-A1- 2008 293 661
- US-A1- 2009 155 255
- US-A1- 2009 155 255
- Banerjee P. et al.: "HTLV-1 infection of human hematopoietic stem cells induces a novel T cell lymphoma in humanized SCID mice.", 50th ASH annual meeting and exposition Blood, vol. 112, no. 11 6 December 2008 (2008-12-06), 16 November 2008 (2008-11-16), page 489, XP002684775, Retrieved from the Internet: URL:ash.confex.com/ash/2008/webprogram/Pap er15202.html [retrieved on 2012-10-08]
- DE BONT E. ET AL.: "Mobilized human CD34+ hematopoietic stem cells enhance tumor growth in a nonobese diabetic/severe combined immunodeficient mouse model of human non-Hodgin's lymphoma.", CANCER RES., vol. 61, 15 October 2001 (2001-10-15), pages 7654-7659, XP002684776,
- LETAI A. ET AL.: "Antiapoptotic BCL-2 is required for mainetnance of a model leukemia", CANCER CELL, vol. 6, September 2004 (2004-09), pages 241-249, XP002684777,
- ZHANG C. ET AL.: "Angiopoietin-like 5 and IGFBP2 stimulate ex vivo expansion of human cord blood hematopoietic stem cells as assayed by NOD-SCID transplantation", BLOOD, vol. 111, 17 January 2008 (2008-01-17), pages 3415-3423, XP002684778,
- LAPALOMBELLA R. ET AL.: "A novel Raji-Burkitt's lymphoma model for preclinical and mechanistic evaluation of CD52-targeted immunotherapeutic agents", CLIN. CANCER RES., vol. 14, no. 2, 15 January 2008 (2008-01-15), pages 569-578, XP002684779,
- GRASSMANN R., ABOUD M., & JEANG K.-T.: "Molecular mechanisms of cellular transformation by HTLV-1 Tax", ONCOGENE, vol. 24, 2005, pages 5976-5985, XP002684780,
- BANERJEE P. ET AL.: "Adult T-cell leukemia/lymphoma development in HTLV-1-infected humanized SCID mice", BLOOD, vol. 115, 12 February 2010 (2010-02-12), pages 2640-2648, XP002684781,
- AGERSTAM H. ET AL.: "Modelling the human 8p11-myeloproliferative syndrome in immunodeficient mice", BLOOD, vol. 116, 16 June 2010 (2010-06-16), pages 2103-2111, XP002684782,
- Leskov I.: "Modeling hematologic malignancies and their treatment in humanized mice", Thesis (Ph. D.)--Massachusetts Institute of Technology, Dept. of Biology, 2010. , 2010, 2010, XP002684783, Retrieved from the Internet: URL:http://dspace.mit.edu/handle/1721.1/61 884?show=full [retrieved on 2012-10-08]
- HOSEN N. ET AL.: "CD96 is a leukemic stem cell-specific marker in human acute myeloid leukemia", PNAS, vol. 104, no. 26, 26 June 2007 (2007-06-26), pages 11008-11013,
- WEIDMANN E. ET AL.: "A phase II study of alezumab, fludrabine, cyclophosphamide and doxorubicin (Campath-FCD) in peripheral T-cell lymphoma.", LEUKEMIA & LYMPHOMA, vol. 51, no. 3, March 2010 (2010-03), pages 447-455,
- HAHN WILLIAM C ET AL: "Rules for making human tumor cells", NEW ENGLAND JOURNAL OF MEDICINE, vol. 347, no. 20, 14 November 2002 (2002-11-14), pages 1593-1603, ISSN: 0028-4793
- LESKOV I ET AL: "Rapid generation of human B-cell lymphomas via combined expression of Myc and Bc12 and their use as a preclinical model for biological therapies", ONCOGENE, vol. 32, no. 8, February 2013 (2013-02), pages 1066-1072, DOI: 10.1038/ONC.2012.117

## Description

### BACKGROUND OF THE INVENTION

Cancer is the second-leading cause of death in the United States, after heart disease; approximately 10% of all cancer deaths were due to neoplasms of the hematopoietic system (Heron, M.P. and B.L. Smith. 2007. National Vital Statistics Reports Vol. 55, No. 10; U.S. Cancer Statistics Working Group. 2006. United States Cancer Statistics 2003 incidence and mortality. U.S. Department of Health and Human Services). These include leukemias, which are disseminated cancers of the hematopoietic stem cells and early progenitors, and lymphomas, which are cancers of mature lymphocytes that arise as discrete masses. In humans, the vast majority (80%-85%) of both aggressive and indolent lymphomas are of B cell origin (Aster, J.C. 2005. Diseases of the White Blood Cells, Lymph Nodes, Spleen, and Thymus. In: Robbins and Cotran Pathologic Basis of Disease, 7th edition (Kumar, V., A.K. Abbas, and N. Fausto, eds.) Elsevier Inc., Philadelphia, PA, pp. 661-709).

The document by Banerjee P et al (2008), Blood, vol.112, nr.11, p.489 describes that HTLV-1 infection of human hematopoietic stem cells induces a novel T cell lymphoma in humanized SCID mice.

The document by Weidmann E et al (2010), Leukemia & Lymphoma, vol.51, nr.3, pp.447-455 describes a phase II study of alemtuzumab, fludarabine, cyclophosphamide, and doxorubicin (Campath-FCD) in peripheral T-cell lymphomas.

Improved models of human neoplasms of the hematpoietic system are needed in order to further study and identify treatments for cancer.

### SUMMARY OF THE INVENTION

The invention in its broadest sense is as defined in the independent claims.

In one aspect, the invention is directed to a method of producing a non-human mammal that is a model for a human hematopoietic cancer comprising introducing human hematopoietic stem cells (HSCs) genetically engineered to express the human oncogenes *bcl-2* and *myc* that are associated with human hematopoietic cancer into an immunodeficient non-human mammal. The mammal is maintained under conditions in which the non-human mammal's blood cell lineage is reconstituted by the human HSCs and the oncogenes are expressed in the mammal, thereby producing a non-human mammal that is a model for a human hematopoietic cancer.

In another aspect, the methods of the invention can further comprise serially transplanting the hematopoietic cancer of the non-human mammal (*i.e.,* the humanized non-human mammal model that is a model for a human hematopoietic cancer; the primary humanized non-human mammal model) to other non-human mammals, thereby producing one or more additional non-human mammals that are models for a human hematopoietic cancer (secondary humanized non-human mammal model; subsequent humanized non-human mammal model(s)). In this aspect, the method comprises introducing human cells that express the human oncogenes *bcl-2* and *myc* from the humanized non-human mammal that is a model for a human hematopoietic cancer (*e.g*., human hematopoietic cancer cells obtained from the humanized non-human mammal) into one or more immunodeficient non-human mammals. The one or more non-human mammals are maintained under conditions in which the non-human mammal's blood cell lineage is reconstituted by the human HSCs and the oncogenes are expressed in the non-human mammals (the secondary humanized non-human mammal model), thereby producing one or more additional non-human mammals that are models for a human hematopoietic cancer.

The invention is also directed to non-human mammals produced by the methods.

In other aspects, the non-human mammals provided herein can be used to identify one or more agents or treatment protocols (regimens) that can be used to treat a human hematopoietic cancer. In this aspect, the method comprises administering the one or more agents or treatment regimens to a (one or more) non-human mammal described herein. Whether the cancer in the non-human mammal is alleviated is then determined, wherein if the cancer in the non-human mammal is alleviated in the non-human mammal, then the one or more agents or treatment regimens can be used to treat the human hematopoietic cancer. In a particular embodiment, the non-human mammal that is a model used in this method can be one that is a model for a particular human hematopoietic cancer patient. Thus, the one or more agents or treatment protocols are specific for that cancer patient.

In yet another aspect, the invention is directed to a combination consisting of an effective amount of an anti-CD52 antibody and cyclophosphamide for use in treatment of leukemia in an individual. In a particular aspect, the anti-CD52 antibody is alemtuzumab. In yet another aspect, the cancer cell load in bone marrow of the individual is reduced 10,000-fold after administration of alemtuzumab and cyclophosphamide.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

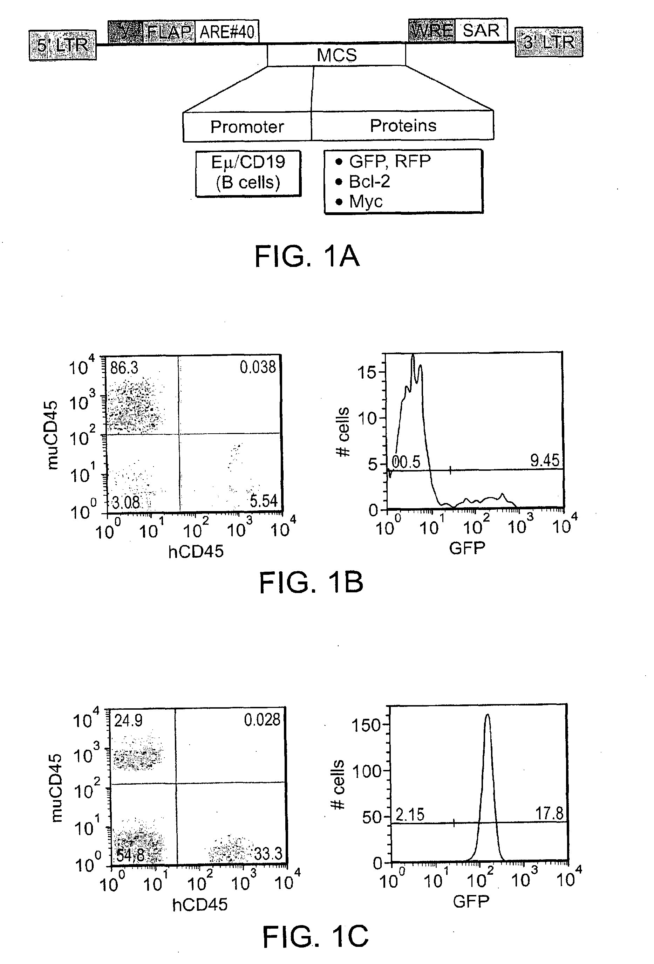

Figures 1A-1C: (Fig. 1A) Lentiviral construct overexpressing GFP, c-Myc and bcl-2 under the control of the Em-enhancer and the CD 19 promotor. Human cord blood derived HSC were infected at MOI of>10 and injected to sublethally irradiated NOD-SCID IL-2Rgamma-/- mice. Engraftment was detected by flow cytometry detection of murine versus human CD45 expression. GFP-Control vector was present in 5% of human cells indicating B-cells derived from successfully infected HSC (Fig. 1B). GFP/*myc*/*bcl* (also refereed to herein as the "GMB model" or the "ALL model) infected HSC reveal >95% of human cells GFP positive (Fig. 1C).

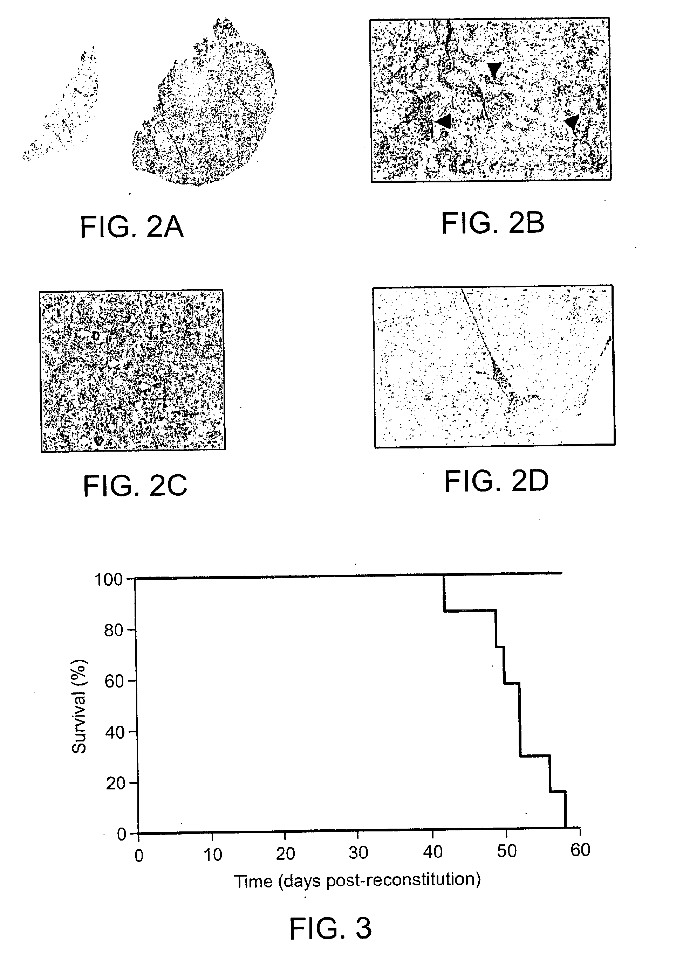

Figures 2A-2D: Histomorphology of humanized GFP/*myc*/*bcl*-Leukemia: Massive infiltration and enlargement of the spleen (Fig. 2A, Fig. 2C); Infiltration of kidneys (Fig. 2B) and brain (Fig. 2D); Infiltrating lymphocytes exhibit blas-like morphology and frequent mitotic figures (Fig. 2C, indicated by arrows).
Figures 3: Cumulative survival of humanized mice after reconstitution with infected HSC. GFP control revealed no impairment of survival (blue line). GFP/*myc*/*bcl* infection of HSC limited survival to max. 2 months following the transfer (red line).

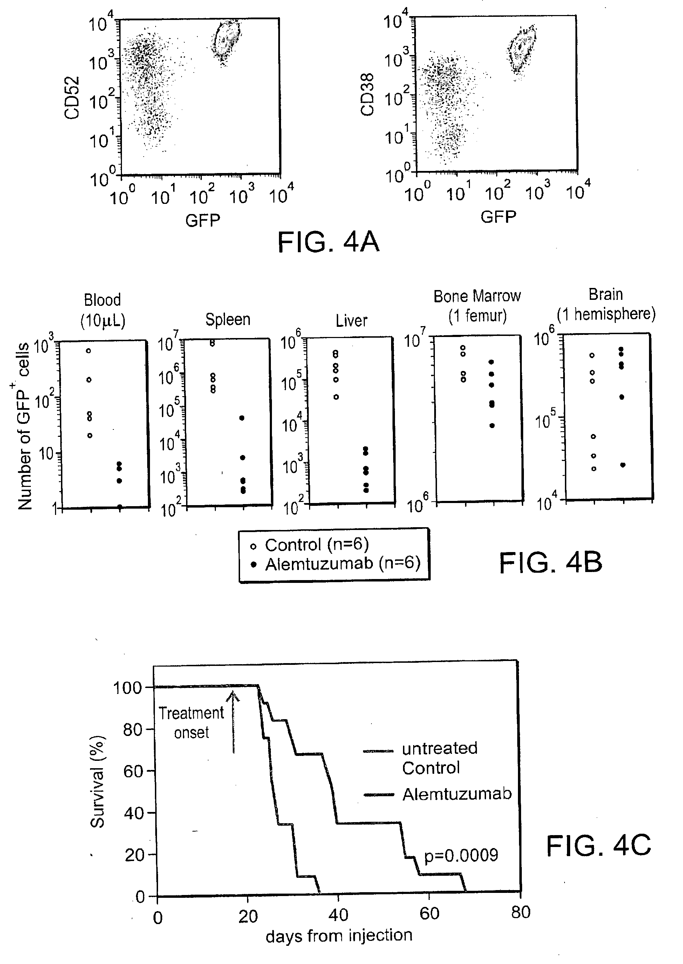

Figure 4A: Expression of putative therapeutic targets CD38 and CD52. Lymphocytes derived from whole spleens were stained for human CD45-PE and CD38-APC or CD52-APC, respectively. Human lymphocytes were gated from FSC/SSC lymphocyte gate and positive hCD45-PE expression. GFP+ GFP/*myc*/*bcl* cells showed uniquely high expression of tumor antigens. GFP-negative non-malignant human cells partly exhibit CD38 and CD52 expression.
Figure 4B: Alemtuzumab treatment of secondary GFP/*myc*/*bcl*-mice: 12NSG-mice were transplanted with 10⁶ GFP/*myc*/*bcl* cells derived from a primary GFP/*myc*/*bcl*-donor mouse. Mice were treated 14 days after transplantation while showing leukemic phenotype. Mice were treated with 5 mg/kg Alemtuzumab (n=6) i.v. or PBS respectively (n=6). Response to treatment was assessed after 7 days, a leukemic burden was measured by flow cytometry and calculating absolute cell count per organ. Alemtuzumab induced significant response in peripheral blood, spleen and liver, while no significant response was seen in bone marrow and brain.
Figure 4C: Cumulative survival for Alemtuzumab treatment (n=10) vs. Control (n=10). Log-rank test revealed significant prolonged survival in alemtuzumab treated mice.

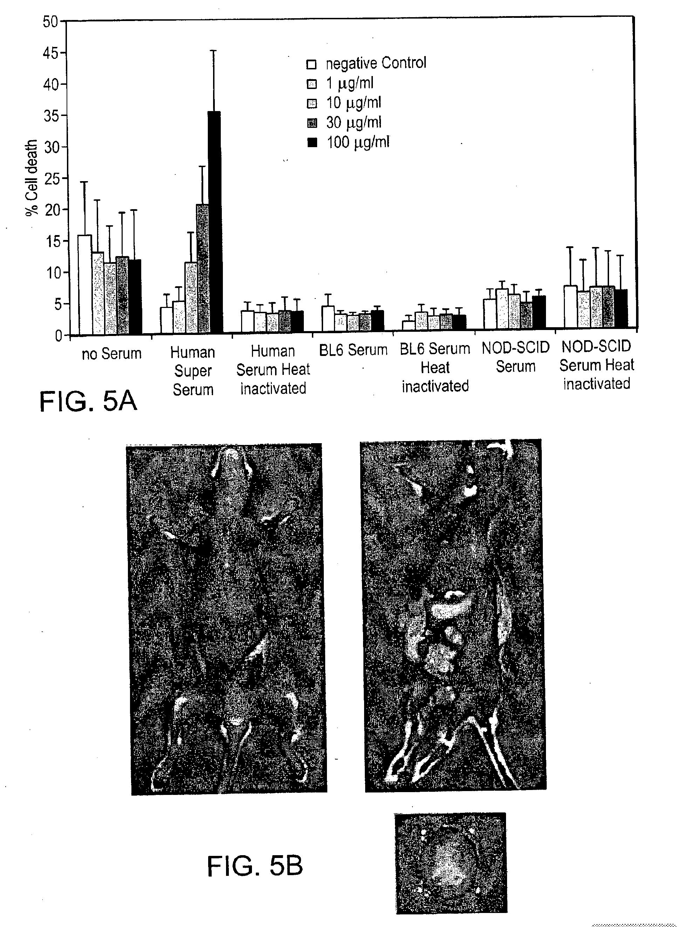

Figure 5A: Complement-dependent cytotoxicity (CDC): GFP/*myc*/*bcl* cells were incubated in vitro with increasing amounts of Alemtuzumab from 1-100 µg/ml. Media were supplemented with human, murine BL6- as NSG-derived serum. Serum-free media and heat-inactivated serum served as control. Cell viability was assessed by Annexin-V-PE/7-AAD flow cytometry after 48 h. Complement-dependent cytotoxicity was detected in human serum, but not in murine sera.
Figure 5B: Alemtuzumab distribution in vivo. AlexaFluor750 labeled Alemtuzumab was injected into GFP/*myc*/*bcl* mice i.v.. Mice were sacrificed after 24 h and distribution of labeled antibody assessed be IVIS fluorometry. Intense staining of spleen, liver and bones. High signal in dissected whole brain.

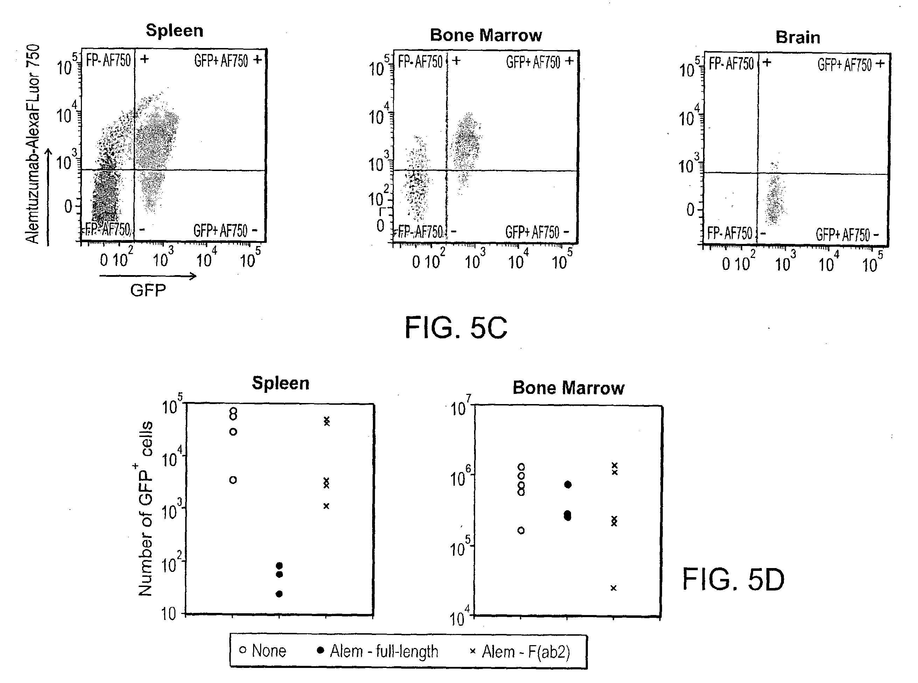

Figure 5C: In vivo binding of GFP/*myc*/*bcl* cells by 5mg/kg Alexafluor750 labeled Alemtuzumab. Organs were homogenized and antibody binding to cells detected by flow cytometry. GFP+ leukemia cells showed increased AlexaFLuor750 signal in spleen and bone marrow, while no detection could be detected in brain-derived leukemia cells.
Figure 5D: Fc-fragment dependent cytotoxic effects of Alemtuzumab. F(ab)2 fragments of Alemtuzumab were injected to secondary GFP/*myc*/*bcl* mice on day 14 after transplantation. F(ab)2-fragments revealed no therapeutic effects vs. Alemtuzumab control.

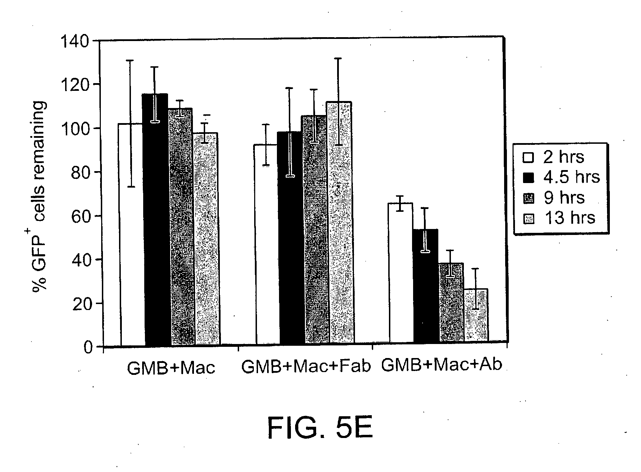

Figure 5E: Antibody-dependent cellular cytotoxicity of Alemtuzumab was assessed using thioglycollate induced macrophages from NSG mice in vitro. Macrophages killed GMB cells within 13 h in the presence of Alemtuzumab. No GFP/*myc*/*bcl*-directed killing was seen for F(ab)2 fragments and macrophage without antibody addition.

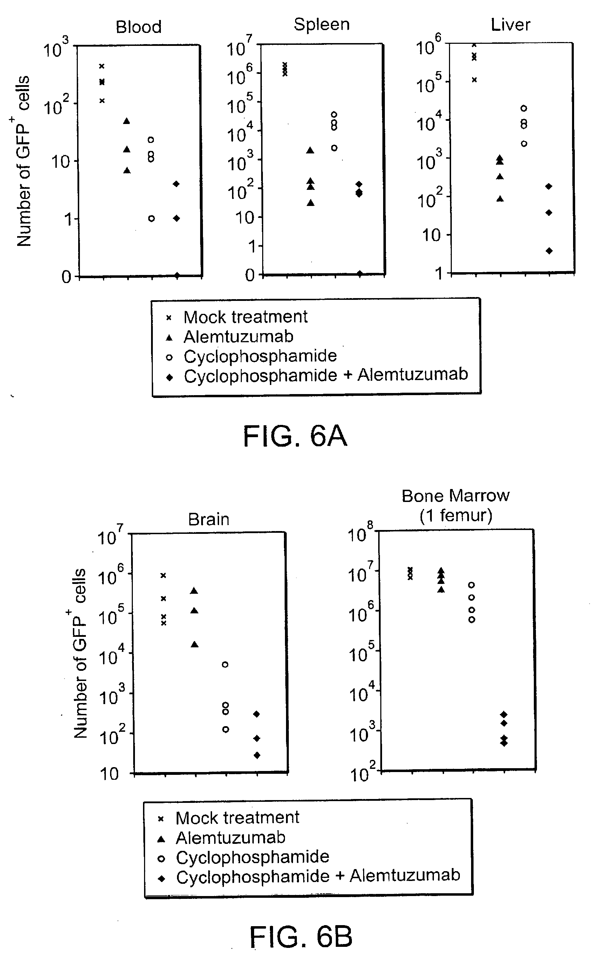

Figures 6A-6B: Combinatorial treatment of Alemtuzumab and cyclophosphamide was performed in secondary GFP/*myc*/*bcl* on day 14 post transplantation. Mice were stratified to no treatment (n=5), 150 mg/kg cyclophosphamide i.p. on days 1 and 2 (n=5), and 5 mg/kg Alemtuzumab i.v. on days 1, 4 and 8. In addition, a combination group received both 150 mg/kg cyclophosphamide i.p. on days 1 and 2 as well as 5 mg/kg Alemtuzumab i.v. on days 1, 4 and 8 (n=5). Absolute number of GFP+ leukemic cells per organ was assessed by FACS and absolute count calculation. Combinatorial treatment shows highest reduction of leukemic cells in peripheral blood, spleen and liver (Fig. 6A). In Alemtuzumab-treatment resistant organs combinatorial treatment revealed highly significant reduction of leukemic cells in bone marrow (Fig. 6B).

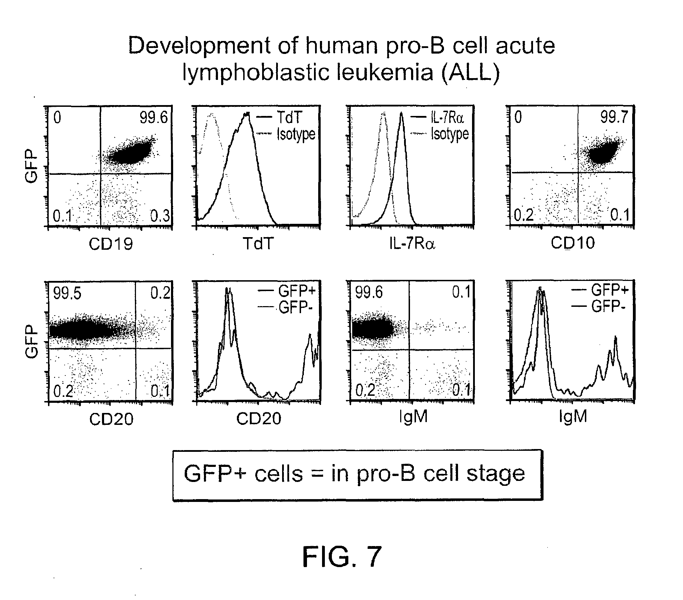

Figure 7 show the development of human pro-B cell acute lymphoblastic leukemia (ALL).

### DETAILED DESCRIPTION OF THE INVENTION

The invention in its broadest sense is as defined in the independent claims.

Hematopoietic (*e.g*., lymphoid; myeloid) malignancies, also referred to herein as hematopoietic cancers, are cancers that originate in the body's hematopoietic tissue (e.g., lymphoid tissue; meloid tissue). A particular genetic mutation known as t(14;18) is frequently found in lymphoid cancers. This mutation causes a cell to produce too much of a protein called Bcl-2, which inhibits the process of programmed cell death (apoptosis) and contributes to tumor formation, tumor growth, and resistance to treatment.

Follicular lymphoma (FL) is the most common of the indolent lymphomas and accounts for 20-40% of all newly-diagnosed cases of non-Hodgkin's lymphoma. The tumor is composed of numerous B cells arranged in lymph node follicles; these B cells are morphologically and phenotypically similar to lymphocytes of a normal germinal center. In the majority of cases (∼85%), FL cells are positive for the t(14;18) chromosomal translocation that links the *bcl-2* oncogene with the immunoglobulin heavy chain (IgH) locus. These cells thus stain strongly positive for the Bcl-2 protein, unlike normal hyperplastic germinal center B cells that are Bcl-2 negative. The lymphoma typically presents as painless swelling of peripheral lymph nodes in middle-aged and elderly individuals, and usually progresses very slowly, with the mean survival of over 10 years. Eventually, however, the progressing lymphadenopathy and lymphocyte infiltration interfere with normal organ function and cause death, although in many patients (up to 70%) end-stage disease is characterized by a histologic transformation to an aggressive diffuse B cell lymphoma that is fatal (Freedman, A.S. and N.L. Harris. 2007. Clinical and pathologic features of follicular lymphoma. In: UpToDate (Rose, B.D., ed.), UpToDate, Waltham, MA; Lossos, I.S. and R. Levy. 2003. Semin. Cancer Biol. 13: 191-202).

However, when the B cell-specific t(14;18) translocation typical of human FL is modeled in mice by placing ectopic *bcl-2* expression under the control of IgH enhancers such as Eµ, the transgenic animals exhibit B cell hyperplasia without lymphoma. Moreover, the hyperplastic B cells in these mice are polyclonal and express only IgM and IgD, whereas in human FL they belong to a single clone, and 40% of them express IgG (Freedman, A.S. and N.L. Harris. 2007. Clinical and pathologic features of follicular lymphoma. In: UpToDate (Rose, B.D., ed.), UpToDate, Waltham, MA; McDonnel, T.J., et al., 1989. Cell 57:79-88; Strasser, A., et al. 1990. Curr. Top. Microbiol. Immunol. 166: 175-181). Eventually the transgenic mice do develop lymphomas (with an average latency of ∼15 months), but unlike FL these are highly aggressive, and ∼50% of them show a secondary rearrangement of the myc gene with the IgH locus (very rare in FL transformation) (McDonnell, T.J. and S.J. Korsmeyer. 1991. Nature 349: 254-256; Kuppers, R., et al. 1999. N. Engl. J. Med. 341(20):1520-1529).

Overexpression of *bcl-2* in murine B cells thus does not appear to be sufficient for generation of follicular lymphoma. A disease much more similar to FL, however, was recently produced in transgenic mice where ectopic *bcl-2* expression was driven by the pan-hematopoietic promoter VavP (Egle, A., et al. 2004. Blood 103: 2278-2283). Healthy young VavP-*bcl*-2 mice develop enlarged germinal centers by 18 weeks of age, replete with hyperplastic B cells, of which 30-50% had undergone class switching and express IgG. By 18 months, however, ∼50% of these mice go on to develop a monoclonal B cell lymphoma, presenting with enlarged lymph nodes and spleen that contain a mixture of centrocytes and mitotic centroblasts. Because *bcl-2* expression in these mice was not restricted to B cells, a significant (3-5-fold) increase in T cells was also seen, especially CD4+ T cells vital for B cell activation in the germinal center. Notably, when VavP-bcl-2 mice were crossed with mice that specifically lack CD4⁺ T cells, the resulting bitransgenic progeny showed no germinal center disease, underscoring the importance of CD4+ T cell help in this disease (Egle, A., et al. 2004. Blood 103: 2278-2283).

While several features of the disease arising in VavP-*bcl*-2 mice do differ from those of human follicular lymphoma (for example, the expanded T cell population), the most important disadvantage of this FL model is its murine nature. In other words, it is unclear that the disease in these mice arises by the same mechanisms as it does in humans, and consequently whether potential drug targets identified in these mice would be relevant in FL patients. Furthermore, the most recent advances in lymphoma treatment involve using monoclonal antibodies directed against the human B-cell specific CD20 antigen. The safety and efficacy of this and other antibodies directed against human antigens, as well as of other, small molecule-type modulators of the immune system, cannot be tested in murine disease models. Creating a humanized mouse model of this and other diseases offers precisely these opportunities.

Described herein are methods of producing a humanized non-human mammal that is a model for a human hematopoietic cancer. As used herein, "humanized non-human mammals (*e.g*., "humanized mice") are immunodeficient non-human mammals (*e.g.,* mice) engrafted with human cells (*e.g.,* hematopoietic cells) or tissues, and/or transgenically express human genes. Such humanized non-human mammals are used, for example, as models of the human immune system and its diseases.

In one aspect, the invention is directed to a method of producing a non-human mammal that is a model for a human hematopoietic cancer comprising introducing human hematopoietic stem cells (HSCs) genetically engineered to express the human oncogenes *bcl-2* and *myc* that are associated with human hematopoietic cancer into an immunodeficient non-human mammal. The mammal is maintained under conditions in which the non-human mammal's blood cell lineage is reconstituted with, or by, the human HSCs (*e.g*., the human HSCs have reconstituted the non-human mammal's blood cell lineage with human blood cell lineages) and the oncogenes are expressed in the mammal, thereby producing a non-human mammal that is a model for a human hematopoietic cancer. As will be appreciated by those of skill in the art, reconstitution of the non-human mammal's blood cell lineage by the human HSCs can be a complete, substantially complete or partial reconstitution.

As discussed above, hematopoietic (*e.g*., lymphoid; myeloid) malignancies, also referred to herein as hematopoietic cancers, are cancers that originate in the body's hematopoietic tissue. As appreciated by those of skill in the art, hematopoietic tissue includes lymphoid and myeloid tissue.

Examples of lymphoid cancers include follicular lymphoma, acute lymphocytic leukemia (ALL), in particular T cell ALL, pro B ALL, pre B ALL, and naive B ALL. Examples in the group of Non-Hodgkin's lymphoma include chronic lymphocytic leukemia (CLL), Burkitt's Lymphoma, diffuse large B cell lymphoma (DLBCL), and mantle cell lymphoma (MCL). Examples of myeloid cancers include acute myeloid leukemias (AML); myelodysplastic syndromes (MDS), chronic myeloid leukemia (CML) or other myeloproliferative diseases (e.g., osteomyelofibrosis, polycythemia vera and essential thrombocythemia).

As used herein, HSCs (*e.g*., human HSCs) are self renewing stem cells that, when engrafted into a recipient, can "repopulate" or "reconstitute" the hematopoietic system of a graft recipient (*e.g*., a non-human mammal; an immunodeficient non-human mammal) and sustain (*e.g*., long term) hematopoiesis in the recipient. The hematopoietic system refers to the organs and tissue involved in the production of the blood cell lineages (*e.g*., bone marrow, spleen, tonsils, lymph nodes). HSCs are multipotent stem cells that give rise to (differentiate into) blood cell types including myeloid cell lineages (*e.g*., monocytes and macrophages, neutrophils, basophils, eosinophils, erythrocytes, megakaryocytes/platelets, dendritic cells) and lymphoid cell lineages (*e.g.,* T-cells, B-cells, NK-cells).

HSCs express the cell marker CD34, and are commonly referred to as "CD34+". As understood by those of skill in the art, HSCs can also express other cell markers, such as CD133 and/or CD90 ("CD133+", "CD90+"). In some instances, HSCs are characterized by markers that are not expressed, *e.g*., CD38 ("CD38-"). Thus, in one embodiment of the invention, the human HSCs used in the methods described herein are CD34+, CD90+, CD133+, CD34+CD38-, CD34+ CD90+, CD34+CD133+CD38-, CD133+CD38-, CD133+CD90+CD38-, CD34+CD133+CD90+CD38-, or any combination thereof. In a particular embodiment, the HSCs are both CD34 ("CD34+") and CD133+ ("CD133+"), also referred to herein as "double positive" or "DP" cells or "DPC". In another embodiment, the HSCs are CD34+CD133+ and CD38- and/or CD90+.

HSCs are found in bone marrow such as in femurs, hip, ribs, sternum, and other bones of a donor (*e.g*., vertebrate animals such as mammals, including humans, primates, pigs, mice, *etc.*). Other sources of HSCs for clinical and scientific use include umbilical cord blood, placenta, fetal liver, mobilized peripheral blood, non-mobilized (or unmobilized) peripheral blood, fetal liver, fetal spleen, embryonic stem cells, and aorta-gonad-mesonephros (AGM), or a combination thereof.

As will be understood by persons of skill in the art, mobilized peripheral blood refers to peripheral blood that is enriched with HSCs (e.g., CD34+ cells). Administration of agents such as chemotherapeutics and/or G-CSF mobilizes stem cells from the bone marrow to the peripheral circulation. For example, administration of granulocyte colony-stimulating factor (G-CSF) for at least, or about, 5 days mobilizes CD34+ cells to the peripheral blood. A 30-fold enrichment of circulating CD34+ cells is observed with peak values occurring on day 5 after the start of G-CSF administration. Without mobilization of peripheral blood, the number of circulating CD34+ cells is very low, estimated between 0.01 to 0.05% of total mononuclear blood cells.

The human HSCs for use in the methods can be obtained from a single donor or multiple donors. In addition, the HSCs used in the methods described herein can be freshly isolated HSCs, cryopreserved HSCS, or a combination thereof.

As known in the art, HSCs can be obtained from these sources using a variety of methods known in the art. For example, HSCs can be obtained directly by removal from the bone marrow, e.g., in the hip, femur, *etc.,* using a needle and syringe, or from blood following pre-treatment of the donor with cytokines, such as granulocyte colony-stimulating factor (G-CSF), that induce cells to be released from the bone marrow compartment.

The HSCs for use in the methods of the invention can be introduced into the non-human mammal directly as obtained *(e.g.,* unexpanded) or manipulated (*e.g.,* expanded) prior to introducing the HSCs into the non-human mammal. In one embodiment, the HSCs are expanded prior to introducing the HSCs into the non-human mammal. As will be appreciated by those of skill in the art there are a variety of methods that can be used to expand HSCs (see *e.g.,* Zhang, Y., et al., Tissue Engineering, 12(8):2161-2170 (2006); Zhang CC, et al., Blood, 111(7):3415-3423 (2008)). In a particular embodiment, a population of HSCs can be expanded by coculturing the HSCs with mesenchymal stem cells (MSCs) in the presence of growth factors (e.g., angiopoietin-like 5 (Angplt5) growth factor, IGF-binding protein 2 (IGFBP2), stem cell factor (SCF), fibroblast growth factor (FGF), thrombopoietin (TPO), or a combination thereof) to produce a cell culture. The cell culture is maintained under conditions in which an expanded population of HSCs is produced *(e.g.,* see Maroun, K., et al., ISSCR, 7th Annual Meeting, Abstract No. 1401 (July 8-11, 2009) Attorney Docket No. 4471.1000-001, PCT Application PCT/US2010/036664, published as WO 2010/138873, filed May 28, 2010).

As described herein, the HSCs introduced into the non-human mammals are genetically engineered so that the HSCs and/or their progeny (*e.g*., a blood lineage cell reconstituted by the human HSCs such as a B cell progeny) express (*e.g*., overexpress) the human oncogenes *bcl-2* and *myc* that are associated with human hematopoietic cancer.

As will be appreciated by those of skill in the art oncogenes "associated with human hematopoietic cancers" include oncogenes that are directly or indirectly involved with the etiology and/or progression of one or more human hematopoietic cancers. A number of oncogenes associated with human hematopoietic cancer are known to those of skill in the art. Examples of such oncogenes include *ABL, AKT, RAS, BRAC1*, *BRAC2, CBL, CDK4, CDK6, PML,* mutant *IDH1* or *IDH2.* As will also be appreciated by those of skill in the art, fused genes associated with hematopoietic cancers can also be used in the methods. Examples of such fused genes include *BCR-ABL, MLL-AF4, MLLAF10* and *MLL-ENO.*

The HSCs express the two oncogenes *bcl-2* and *myc* that are known to be associated with human lymphomas.

Transgenic animals that express (*e.g*., overexpress) both oncogenes in a B-cell specific manner develop leukocyte tumors within ∼3 weeks and die within 5-6 weeks (Strasser, A., et al. 1990. Nature 348: 331-333; Marin, M.C., et al. 1995. Exp. Cell Res. 217: 240-247). Unlike other oncogenes that promote proliferation, *bcl-2* has been found instead to prevent programmed cell death, by inhibiting the activation of apoptosis mediators such as caspases; *bcl-2* overexpression is a hallmark of follicular lymphoma (Chao, D.T. and S.J. Korsmeyer. 1998. Annu. Rev. Immunol. 16: 395-419; Cory, S., et al. 2003. Oncogene 22: 8590-8607).

The *myc* oncogene, on the other hand, is known to prevent terminal cell differentiation, but also has pro-apoptotic functions; its overexpression is strongly associated with the highly aggressive Burkitt's lymphoma. (Marin, M.C., et al. 1995. Exp. Cell Res. 217: 240-247; Nilsson, J.A. and J.L. Cleveland. 2003. Oncogene 22: 9007-9021; Adams, J.M., et al. 1985. Nature 318: 533-538). Interestingly, bcl-2 has been shown to enhance the tumorigenic potential *of myc,* by blocking *myc*-associated apoptosis without inhibiting *myc*-induced cellular proliferation (Marin, M.C., et al. 1995. Exp. Cell Res. 217: 240-247; Meyer, N., et al. 2006. Semin. Cancer Biol. 16: 275-287).

As shown herein, when human HSCs genetically engineered to express *bcl* and *myc* were introduced into mice, and the mice were maintained under conditions in which the HSCs were reconstituted and the oncogenes were expressed in the mice, human lymphoid tumors developed in the *bcl-2*/*myc* double-transgenic humanized mice.

In addition, the HSCs can be genetically engineered to inhibit the expression of one or more tumor suppressor genes in the HSCs and/or the progeny of the HSCs (*e.g*., the blood lineage cells reconstituted by the human HSCs in the non-human mammal). Thus, in one embodiment, the invention is directed to a method of producing a non-human mammal that is a model for a human hematopoietic cancer further comprising introducing human hematopoietic stem cells (HSCs) that include (comprise) nucleic acid that inhibits the expression of one or more tumor suppressor genes in the HSCs and/or the progeny of the HSCs into an immunodeficient non-human mammal. The mammal is maintained under conditions in which the non-human mammal's blood cell lineage is reconstituted by the human HSCs and expression of the one or more tumor suppressor genes are inhibited in the mammal, thereby producing a non-human mammal that is a model for a human hematopoietic cancer.

In another embodiment, the invention is directed to a method of producing a non-human mammal that is a model for a human hematopoietic cancer comprising introducing human hematopoietic stem cells (HSCs) that include (comprise) nucleic acid that encodes (*e.g*., genetically engineered to express) the human oncogenes *bcl-2* and *myc* that are associated with human hematopoietic cancer, and nucleic acid that inhibits the expression of one or more tumor suppressor genes, in the HSCs and/or in the progeny of the HSCs into an immunodeficient non-human mammal. The mammal is maintained under conditions in which the non-human mammal's blood cell lineage is reconstituted by the human HSCs and the oncogenes are expressed, and expression of the one or more tumor suppressor genes are inhibited in the mammal, thereby producing a non-human mammal that is a model for a human hematopoietic cancer.

Tumor suppressor genes express proteins that help prevent - or "suppress" - abnormal cells from developing into full-blown tumors. When such genes are disabled, as they frequently are in cancer cells, cells can grow uncontrollably, forming tumors that are the hallmarks of cancer. Examples of tumor suppressor genes include *p*53, *Rb, APC, PTEN, CD95, ATM,* and *DAPKI.*

As will be appreciated by those of skill in the art, any suitable nucleic acid that inhibits and/or reduces expression of one or more tumor suppressor genes can be used in the methods of the invention. For example nucleic acid that knocks out, knocks in and/or knocks down the expression of a tumor suppressor gene can be used in the methods of the invention. An example of such nucleic acid includes nucleic acid that is targeted for insertion within a tumor suppressor gene thereby deleting or interrupting the expression of (knocking down) the tumor suppressor gene. Another example is nucleic acid that is targeted to replace the tumor suppressor gene (*e.g*., nucleic acid that encodes a gene other than a tumor suppressor gene) thereby knocking in the tumor suppressor gene. Further examples include nucleic acid with a sequence complementary to the tumor suppressor gene or an mRNA transcript of the tumor suppressor gene which results in decreased expression through blocking of transcription (in the case of gene-binding), degradation of the mRNA transcript (e.g. by small interfering RNA (siRNA) or RNase-H dependent antisense) or blocking either mRNA translation, pre-mRNA splicing sites or nuclease cleavage sites used for maturation of other functional RNAs such as miRNA (Summerton, J (2007), Med Chem., 7(7):651-660) (e.g. by Morpholino oligos or other RNase-H independent antisense (Summerton, J., (1999) Biochimica et Biophysica Acta 1489 (1): 141-58).

As will be appreciated by those of skill in the art, HSCs can be genetically engineered to express *(e.g.,* overexpress) the human oncogenes *bcl-2* and *myc,* fused genes or tumor suppressor genes using a variety of techniques known in the art. For example expression of nucleic acid encoding the human oncogenes *bcl-2* and *myc,* and/or nucleic acid that inhibits expression of one or more tumor suppressor genes, by the HSCs can be attained by transducing human HSCs using one or more suitable vectors that comprises the nucleic acid. In one embodiment, the vectors are viral vectors. Examples of suitable viral vectors for use in the methods described herein include adenovirus, adeno-associated virus, lentivirus, retrovirus and the like.

As also appreciated by those of skill in the art, the nucleic acid encoding the human oncogenes *bcl-2* and *myc* and/or nucleic acid that inhibits expression of one or more tumor suppressor genes can be placed in a single vector or in multiple vectors, along with a reporter/selection marker. Examples of suitable reporter/selection markers include GFP, RFP, and other fluorescent proteins (*e.g*., mCherry, Tomato, cyan, luciferase) and cell surface markers (*e.g*., Thy-1, antibiotic resistance genes). Furthermore, the human oncogenes *bcl-2* and *myc* and/or nucleic acid that inhibits expression of one or more tumor suppressor genes can be expressed from a single promoter in stoichiometric amounts. In particular embodiments when more than one oncogene and/or nucleic acid that inhibits expression of one or more tumor suppressor genes is used, the oncogenes and/or nucleic acid that inhibits expression of one or more tumor suppressor genes can be separated by a region encoding a ribosome-skip-inducing peptide such as the 2A peptide (Szymczak, A.L., et al. 2004. Nat. Biotechnol. 22: 589-594; Fang, J., et al. 2005. Nat. Biotechnol. 23: 584-590).

Vectors comprising the human oncogenes *bcl-2* and *myc* and/or nucleic acid that inhibits expression of one or more tumor suppressor genes can further comprise a variety of promoters operably linked to the human oncogenes *bcl-2* and *myc.* For example, the human oncogenes *bcl-2* and *myc* and/or nucleic acid that inhibits expression of one or more tumor suppressor genes can be operably linked to an Eµ enhancer plus CD 19 promoter or an EF1α promoter; the former will primarily direct expression of the oncogenes and/or nucleic acid that inhibits expression of one or more tumor suppressor genes in B cells, while the latter will direct expression of the oncogenes and/or nucleic acid that inhibits expression of one or more tumor suppressor genes in many blood lineages.

The vectors can comprise additional elements known to those of skill in the art. For example, the vector can further comprise an IRES-driven reporter. In addition, as described herein, viral pseudotype can be used to further optimize infection. For example, viruses (e.g., lentivirus) psuedotyped with the envelope protein RD114, the surface glycoprotein VSV-G (Brenner, S. and H.L. Malech. 2003. Biochim. Biophys. Acta. 1640: 1-24; Sandrin, V., et al. 2002. Blood 100: 823-832; Di Nunzio, et al. 2007. Hum. Gene Ther. 18: 811-20), or Gibbon ape leukemia virus (GAVL) coat protein can be used.

In the methods of the invention, the HSCs engineered to express the human oncogenes *bcl-2* and *myc* are introduced into a non-human mammal. As used herein, the terms "mammal" and "mammalian" refer to any vertebrate animal, including monotremes, marsupials and placental, that suckle their young and either give birth to living young (eutharian or placental mammals) or are egg-laying (metatharian or nonplacental mammals). Examples of mammalian species that can be used in the methods described herein include non-human primates (e.g., monkeys, chimpanzees), rodents (e.g., rats, mice, guinea pigs), canines, felines, and ruminents (e.g., cows, pigs, horses). In one embodiment, the non-human mammal is a mouse. The non-human mammal used in the methods described herein can be adult, newborn *(e.g.,* < 48 hours old; pups), or *in utero.*

The non-human mammal is an immunodeficient non-human mammal, that is, a non-human mammal that has one or more deficiencies in its immune system *(e.g.,* NSG or NOD scid gamma (NOD.*Cg-Prkdcscid Il2rgtm1 Wjl*/*SzJ*) mice) and, as a result, allow reconstitution of human blood cell lineages by the human HSCs when introduced. For example, the non-human mammal lacks its own T cells, B cells, NK cells or a combination thereof. In particular embodiments, the non-human mammal is an immunodeficient mouse, such as a non-obese diabetic mouse that carries a severe combined immunodeficiency mutation (NOD/scid mouse); a non-obese diabetic mouse that carries a severe combined immunodeficiency mutation and lacks a gene for the cytokine-receptor γ chain (NOD/scid IL2R γ-/- mouse) and a Balb/c rag-/- γc-/-mouse.

Other specific examples of immunodeficient mice include, but are not limited to, severe combined immunodeficiency (*scid*) mice, non-obese diabetic (NOD)-*scid* mice, *IL2rg*^{*-*/*-*} mice (*e.g.,* NOD/LySz-*scid IL2rg*^{*-*/*-*} mice, NOD/Shi- *scid IL2rg*^{*-*/*-*} mice (NOG mice), BALB/c- *Rag*^{*-*/}*⁻IL2rg*^{*-*/*-*} mice, H2^{d}-*Rag*^{*-*/}*⁻IL2rg*^{*-*/*-*} mice), NOD/*Rag*^{*-*/}*⁻IL2rg*^{*-*/*-*} mice.

Non-obese diabetic (NOD) mice carrying the severe combined immunodeficiency (scid) mutation are currently the most widely-used xenotransplant recipients. The engraftment of human cells in these NOD/scid mice, however, still does not exceed several percent, probably because of the residual presence of innate immunity and the low but present NK-cell activity in these mice (Shultz, L.D., et al. 2007. Nat. Rev. Immunol. 7: 118-130; Chicha L., R. et al. 2005. Ann. N. Y. Acad. Sci. 1044:236-243). Their usefulness is further limited by their high predisposition to thymic lymphomas and thus a relatively short life span (∼37 weeks) (Shultz, L.D., et al. 2005. J. Immonol. 174: 6477-6489).

Recently, Shultz *et al.* generated NOD/scid mice that were lacking the gene for the common cytokine-receptor γ chain, a vital subunit of receptors for various cytokines crucial for lymphoid development (Shultz, L.D., et al. 2005. J. Immonol. 174: 6477-6489; Cao, X., et al. 1995. Immunity 2: 223-238). The resulting NOD/scid, γc^{null} mice are free of thymic lymphomas, have a much longer life span (∼90 weeks), and have more profound deficiencies in their innate immunity than the NOD/scid mice; consequently they permit >10-fold greater engraftment of human cells in their bone marrow (∼70% of cells in their bone marrow are human, vs. ∼6% in NOD/scid mice) (Shultz, L.D., et al. 2005. J. Immonol. 174: 6477-6489; Ishikawa, F., et al. 2005. Blood 106(5): 1565-1573).

Human HSCs in these mice gave rise to B cell precursors and mature IgM⁺ B cells in the bone marrow, as well as NK cells, myeloid cells, dendritic cells, and stem cells. The thymus contained T cell precursors, and peripheral blood leukocytes were primarily CD4⁺ and CD8⁺ T cells. The majority of splenocytes were human B cells arranged in follicular structures; soluble human IgM and IgG were detected in the peripheral blood, indicating the occurrence of class switching. Finally, follicle-like structures containing mostly B cells surrounding some T cells were observed in the spleen and mesenteric lymph nodes, and B cells were shown to be able to produce antigen-specific antibodies (both IgM and IgG) after immunization with ovalbumin (Shultz, L.D., et al. 2005. J. Immonol. 174: 6477-6489; Ishikawa, F., et al. 2005. Blood 106(5): 1565-1573).

In some embodiments, the non-human mammal is treated or manipulated prior to introduction of the HSCs engineered to express the human oncogenes *bcl-2* and *myc (e.g.,* to further enhance reconstitution of the human HSCs). For example, the non-human mammal can be manipulated to further enhance engraftment and/or reconstitution of the human HSCs. In one embodiment, the non-human mammal is irradiated prior to introduction of the HSCs and the (one or more) nucleic acid encoding the one or more cytokines. In another embodiment, one or more chemotherapeutics are administered to the non-human mammal prior to introduction of the HSCs.

As will also be appreciated by those of skill in the art, there are a variety of ways to introduce HSCs engineered to encode the human oncogenes *bcl-2* and *myc* into a non-human mammal. Examples of such methods include, but are not limited to, intradermal, intramuscular, intraperitoneal, intraocular, intrafemoral, intraventricular, intracranial, intrathecal, intravenous, intracardial, intrahepatic, intra-bone marrow, subcutaneous, topical, oral and intranasal routes of administration. Other suitable methods of introduction can also include, *in utero* injection, hydrodynamic gene delivery, gene therapy, rechargeable or biodegradable devices, particle acceleration devises ("gene guns") and slow release polymeric devices. The HSCs can be introduced into the non-human using any such routes of administration or the like.

In the methods of the invention, after the human HSCs engineered to express the human oncogenes *bcl-2* and *myc* are introduced into the non-human mammal, the non-human mammal is maintained under conditions in which the non-human mammal is reconstituted with the human HSCs and the oncogenes are expressed in the mammal. Such conditions under which the non-human animals of the invention are maintained include meeting the basic needs (*e.g*., food, water, light) of the mammal as known to those of skill in the art. In particular embodiments, maintaining the animal under such suitable conditions results in the development of lymphoid tumors in the non-human mammal.

The methods of the invention can further comprise determining whether the nucleic acid encoding the one or more cytokines is expressed and/or the non-human is reconstituted with the HSCs. Methods for determining whether the nucleic acid is expressed and/or the non-human mammal's blood cell lineage is reconstituted by the HSCs are provided herein and are well known to those of skill in the art. For example, flow cytometry analysis using antibodies specific for surface cell markers of human HSCs can be used to detect the presence of human HSCs or the progeny of the human HSCs in the non-human mammal (*e.g*., the blood lineage cell into which the human HSCs have differentiated in the non-human mammal). In addition, following reconstitution, the general health of recipient mice can be carefully monitored. Such monitoring can include obtaining peripheral white blood cell counts and cell marker phenotype. In particular embodiments, flow cytometry and immunohistochemistry can be used to characterize the cellular composition of the non-human mammal's primary and secondary lymphoid organs. In addition, reconstitution of human blood cell lineages by the human HSCs in the non-human mammal can be assessed by detecting human leukocytes that express the human oncogenes *bcl-2* and *myc* in the non-human mammal.

In another aspect, the methods of the invention can further comprise serially transplanting the lymphoid cancer of the non-human mammal (*i.e*., the humanized non-human mammal model that is a model for a human lymphoid cancer; the primary humanized non-human mammal model) to other non-human mammals, thereby producing one or more additional non-human mammals that are models for a human lymphoid cancer (secondary humanized non-human mammal model). In this aspect, the method comprises introducing human cells that express the human oncogenes *bcl-2* and *myc* from the humanized non-human mammal that is a model for a human lymphoid cancer (*e.g.*, human leukocytes obtained from the humanized non-human mammal) into one or more immunodeficient non-human mammals. The one or more non-human mammals are maintained under conditions in which the human HSCs are reconstituted and the oncogenes are expressed in the second non-human mammal, thereby producing one or more additional non-human mammals that are models for a human lymphoid cancer. In particular embodiments, the additional one or more non-human mammals are the same or a similar species as the original humanized non-human mammal model (*i.e*., the original non-human mammal model is a humanized mouse model and the additional non-human mammal models are mice). In other embodiments, the additional one or more non-human mammals are a different species as the original humanized non-human mammal model.

As will be appreciated by those of skills in the art, several types of cells that express the human oncogenes *bcl-2* and *myc* obtained from the humanized non-human mammal can be used in the method. For example, human cells that express the human oncogenes *bcl-2* and *myc* obtained from the bone marrow or the spleen of the humanized non-human mammal can be used. In a particular embodiment, the cells are splenocytes of the humanized non-human mammal (the primary humanized non-human mammal).

Methods for obtaining (isolating, purifying, substantially purifying) such cells are known to those of skill in the art. As used herein, "isolated" (*e.g*., "isolated cells that express one or more human oncogenes") refers to substantially isolated with respect to the complex (*e.g*., cellular) milieu in which it naturally occurs, or organ, body, or culture medium. In some instances, the isolated material will form part of a composition (for example, a crude extract containing other substances), buffer system or reagent mix. In other circumstances, the material can be purified to essential homogeneity. An isolated cell population can comprise at least about 50%, at least about 80%, at least about 85%, at least about 90%, at least about 95%, or at least about 99% (on a total cell number basis) of all cells present.

In some aspects, the cells that express the human oncogenes *bcl-2* and *myc* obtained from the humanized non-human mammal can be injected directly into one or more non-human mammals. In other aspects, the cell can be expanded as described herein prior to introduction into the non-human mammal(s).

Thus, cohorts (*e.g*., about 2, about 3, about 4, about 5, about 6, about 7, about 8, about 9, about 10, about 20, about 30, about 40, about 50, about 60, about 70, about 80, about 90, about 100 *etc.*) of non-human mammals that are models for a human lymphoid cancer can be produced using human cells that express the human oncogenes *bcl-2* and *myc* obtained from the original (first; primary) non-human mammal that was produced by introducing human HSCs engineered to express the human oncogenes *bcl-2* and *myc* that are associated with human hematopoietic cancer. As will be appreciated by those of skill in the art, these mice also be used to serially transplant one or more cohorts.

The discovery that a humanized non-human mammal that is a model for a human hematopoietic cancer can be produced by introducing human HSCs engineered to express the human oncogenes *bcl-2* and *myc* associated with hematopoietic cancer provides for a variety of uses.

Also described herein is a method of producing a non-human mammal that is a model for a human hematopoietic cancer patient. The method comprises introducing hematopoietic stem cells (HSCs) of the lymphoid cancer patient into an immunodeficient non-human mammal. The non-human mammal is maintained under conditions in which the non-human mammal's blood cell lineage is reconstituted by the human HSCs and oncogenes of the cancer patient are expressed in the non-human mammal, thereby producing a non-human mammal that is a model for the hematopoietic cancer patient.

In other aspect, the invention provides a non-human mammal produced by the methods described herein, as recited in the claims.

In yet another aspect, the non-human mammals provided herein can be used to identify one or agents or treatment regimens (protocols) that can be used to treat a human hematopoietic cancer. In this aspect, the method comprises administering the one or more agents or treatment regimens to a (one or more) non-human mammal described herein. Whether the cancer in the non-human mammal is alleviated is then determined, wherein if the cancer in the non-human mammal is alleviated in the non-human mammal, then the one or more agents or treatment regimens can be used to treat the human lymphoid cancer. In a particular aspect, the non-human mammal that is a model used in this method can be one that is a model for a particular human lymphoid cancer patient. That is, the non-human mammal is one that was produced by introducing HSCs of that particular lymphoid cancer patient. Thus, the one or more agents or one or more treatment protocols are specific for that patient.

As one of skill in the art will appreciate, alleviation of a human lymphoid cancer includes removal of the cancer, remission of the cancer, prolonging the life of the cancer patient, or improving the quality of life of a cancer patient or combinations thereof.

As will also be appreciated by one of skill in the art, the method can further comprise comparing whether the cancer in the non-human mammal is alleviated compared to a variety of suitable controls. An example of such a control is a non-human mammal that has not received the agent or treatment regimen.

As shown herein, the humanized non-human mammal models of lymphoid cancer were used to identify a treatment protocols that produced a synergistic effect in the treatment of lymphoid cancer. Specifically, as described in Example 3, because the surface marker CD52 is expressed in the model of ALL described herein, these cohorts were used to test the efficacy of the monoclonal antibody Alemtuzumab (Campath-1), which targets human CD52 and has been approved by the FDA for use against relapsed Chronic Lymphocytic Leukemia. Also, whether treating mice with the widely used chemotherapeutic agent cyclophosphamide at the start of Alemtuzumab treatment would improve its efficacy was tested. It was found that the combination of cyclophosphamide and Alemtuzumab was slightly (∼5-10-fold) more effective than Alemtuzumab alone in the blood, liver and spleen, and also slightly (∼5-10-fold) more effective than cyclophosphamide alone in the brain. Notably, however, treating mice with a combination of cyclophosphamide and Alemtuzumab decreased their cancer cell load in the bone marrow by ∼10,000-fold or more, so that few, if any, cells were detectable in femoral bone marrow samples after the treatment.

Thus, in another aspect, the invention is directed to a combination consisting of an effective amount of an anti-CD52 antibody and cyclophosphamide for use in treatment of leukemia in an individual. Also described herein is a method of treating leukemia in an individual in need thereof comprising simultaneously administering an effective amount of an anti-CD52 antibody and one or more chemotherapeutic agents to the individual. In a particular aspect, the anti-CD52 antibody is Alemtuzumab and the one or more chemotherapeutic agents is cyclophosphamide. In yet other aspects, the cancer cell load in bone marrow of the individual is reduced about 10-fold, about 20-fold, about 50-fold, about 100-fold, about 150-fold, about 200-fold, about 300-fold, about 400-fold, about 500-fold, about 600-fold, about 700-fold, about 800-fold, about 900-fold, about 1000-fold, about 2000-fold, about 3000-fold, about 4000-fold, about 5000-fold, about 6000-fold, about 7000-fold, about 8000-fold, about 9000-fold, about 10,000-fold, about 20-000-fold after administration of the alemtuzumab and the cyclophosphamide. In a particular aspect, the cancer cell load in bone marrow of the individual is reduced 10,000-fold after administration of the Alemtuzumab and the cyclophosphamide.

### Exemplification

### Example 1 Humanized mouse model of cancer of the immune system.

Described herein is the creation of a humanized mouse models of lymphoid cancers (e.g., leukemias and lymphomas).

Lentiviral backbone was prepared in which a B-cell specific promoter (the human CD 19 promoter combined with the Eµ enhancer) controlled the expression of green fluorescent protein (GFP) and the oncogenes Myc and/or Bcl-2. VSVG-pseudotyped lentivirus was prepared using this backbone, as previously described, and concentrated using ultracentrifugation. Viral titer was determined.

Human CD34⁺ or CD133⁺ cells, derived from fetal tissues or cords, were purified using magnetic sorting and re-suspended in culture media containing 4ug/ml polybrene. Previously prepared lentivirus carrying human oncogenes (see above) was then added, such that the multiplicity of infection (MOI) was 10 or more. The mixture of cells and virus was then aliquoted into a 96-well U-bottom plate, so that each well contained 20,000-50,000 cells. Cells were then centrifuged at 1000xG for 90-120 min. at room temperature (25°C). Subsequently the cells were incubated at 37°C overnight. The following day virus-containing media was replaced by fresh (virus-free) cell culture media, and cells were re-suspended.

The infection protocol described above can also be applied to human CD34+ or CD133⁺ cells that had been expanded in vitro using a previously described culture protocol (Zhang et al, 2008, Blood 111(7) 3415-23). Following expansion, CD133+ cells are re-purified and infected as described above.

Approximately 60 hours following infection, cells were pooled, re-suspended in StemSpan^{®} media, and injected into sublethally irradiated immunodeficient NOD/scid/γc^{null} host mice. The mice were then monitored approximately once every 10 days, both visually and by screening peripheral blood for the appearance of human cells (e.g., positive for human CD45 antigen).

Mice reconstituted with cells expressing GFP as well as both Myc and Bcl-2 oncogenes began experiencing weight loss within 2 months of reconstitution. Over the course of the subsequent 3-5 weeks these mice grew progressively weaker, eventually becoming unable to feed themselves; at the same time, human leukocytes that were GFP+ (and thus also Myc+ and Bcl-2+) increased in number until they made up over 95% of leukocytes in the peripheral blood. At this point the mice were sacrificed and examined. See Figures 1A-1C.

All mice reconstituted with cells expressing GFP, Myc, and Bcl-2 exhibited splenomegaly and bone marrow infiltration, with several also exhibiting enlarged lymph nodes, inflamed lungs, and evidence of tumor invasion into their liver, kidneys, brain and muscles around the spine. See Figures 2A-2D, and Figure 3.

### Example 2 The Cancer of the Immune System was Serially Transplantable

The disease was shown to be serially transplantable into other NOD/scid/ γc^{null} mice. Secondary transplanted mice exhibited phenotypically similar leukemias as the primary donor. Migration pattern of leukemic cells as well as clinical symptoms were similar. Lifespan of these secondary recipients was shown to be dependent on the number of GFP+ cells with which they were injected initially. Cells were purified based on the GFP marker expression by flow cytometry on a MoFlow cell sorter. As an alternative method, magnetic positive selection for CD 19+ cells revealed similar purities of > 99 %. Cells were injected directly after isolation and purification without further expansion in order to avoid in vitro selection of potential subpopulation. When injected with approximately one million GFP+ cells (resuspended in ∼100 uL StemSpan^{®} media), taken from either bone marrow or the spleen of primary host mice, secondary recipients developed an identical leukemia ∼3 weeks after the initial injection, and succumbed to it ∼1-2 weeks afterwards. Using GFP+ splenocytes from primary leukemic mice, large cohorts *(e.g.,* 50-100 animals each) of secondary mice were created. Given the high numbers of malignant cells isolated from spleens *(e.g.,* of up to 10⁹ cells), no relevant limitations in size of secondary transplanted cohorts occur.

### Example 3 Use of the Humanized Model to Investigate Human-Specific Therapeutics

The humanized acute lymphoblastic leukemia (ALL) model offers the opportunity to investigate novel human-specific therapeutic agents, particularly monoclonal antibodies targeting tumor-associated antigens. Leukemic cells in this model express a variety of cell surface markers, some of which are targets for monoclonal antibodies or biologicals that are on the market or in preclincal development such as CD52, CD22, CD19, CD38, CD44, CD47, CD74, DR4, DR5, CD123 and NRP1.

Lymphocytes derived from whole spleens were stained for human CD45-PE and CD38-APC or CD52-APC respectively. Human lymphocytes were gated from FSC/SSC lymphocyte gate and positive hCD45-PE expression. GFP+ GMB cells showed uniquely high expression of tumor antigens. GFP-negative non-malignant human cells partly exhibit CD38 and CD52 expression. See Figure 4A.

Because the surface marker CD52 is expressed in the model of ALL described herein, these cohorts were used to test the efficacy of the monoclonal antibody Alemtuzumab (Campath-1), which targets human CD52 and has been approved by the FDA for use against relapsed Chronic Lymphocytic Leukemia. Each mouse in a cohort of secondary mice was injected with ∼10⁶ GFP+ cells from the same primary donor mouse. 18-20 days later, mice were divided into 2 groups, and stratified for the density of GFP+ cells found in their peripheral blood (i.e. having the same (or similar) number of GFP+ cells/uL of blood). One group of mice then received 3 tail-vein injections of 5mg/kg bodyweight Alemtuzumab, diluted to 1mg/ml in sterile PBS, on days 1,4, and 7 of treatment, starting on the day the mice had been divided into groups; the other group of mice received injections of sterile PBS only. Immediately prior to receiving an injection, each mouse was weighed, and the volume of each injection was adjusted accordingly, so that each mouse received 5uL of Alemtuzumab (or PBS) per gram of body weight.

Two days after completing the course of treatment, several mice were euthanized and the number of GFP+ cells in various organs was determined. It was found that Alemtuzumab-treated mice had strikingly fewer GFP+ cells in their peripheral blood (∼30-fold decrease), liver (∼400-fold decrease), and spleen (∼3300-fold decrease) than mice injected with PBS alone. However, the number of GFP+ cells in the bone marrow or the brain of the Alemtuzumab-treated and control mice was approximately the same, indicating a lack of effect of Alemtuzumab in those organs. Nevertheless, Alemtuzumab-treated mice survived significantly longer than PBS-treated ones. See Figure 4B and 4C.

In order to test the mechanisms of Alemtuzumab-mediated leukemia cell killing pepsin-digested F(ab)2-fragments of Alemtuzumab were used revealing no therapeutic effect, indicating that an Fc-Receptor mediated target-effector cell interaction is a crucial mechanism in Alemtuzumab drug action. Antibody-bound leukemic cells can also be killed by complement-mediated lysis. Because NSG mice are deficient of complement C5, serum from NSG mice did not mediate Alemtuzumab mediated tumor cell killing in vitro (Fig. 5A). Similarly, no direct cytotoxic effects on ALL-cells in vitro could be observed (Fig. 5C). However, induced peritoneal macrophages were capable of ALL-cell lysis in vitro while adding Alemtuzumab. Again, no effect was seen when applying F(ab)2 fragments of Alemtuzumab lacking the Fc part (Fig. 5D).

In order to analyze the role macrophages play in anti-leukemic activity of Alemtuzumab in vivo, macrophages were depleted in tumor-bearing mice prior to treatment, by intravenous or intraperitoneal injection of clodronate containing liposomes. When macrophage-depleted mice were treated with Alemtuzumab 2 days after the injection of clodronate liposomes, no reduction of leukemic cells in the spleen was seen.

The humanized ALL model reveals compartment-specific therapeutic responses, in particular Alemtuzumab resistance in brain and bone marrow. In order to elucidate mechanisms of resistance, tissue distribution and epitope binding of Alemtuzumab in vivo was assessed by labeling Alemtuzumab with AlexaFluor 750. Labeled Alemtuzumab was injected at a therapeutic dose of 5 mg/kg. Mice were sacrificed after 24 h and imaged at an IVIS fluorometer. Fluorescent signal was focused on spleen and bones. Organs were homogenized and cells from these organs were directly assessed for Alemtuzumab-AlexaFluor750 binding by flow cytometry. Strong antibody binding was seen in spleen and bone marrow, while no specific binding was seen in brain-derived leukemia cells. Such Alemtuzumab resistance mechanism in the bone marrow are independent of pharmacokinetic aspects, while cells invading the brain are largely shielded by the blood-brain-barrier. See Figures 5B and 5C.

Also, whether treating mice with the widely used chemotherapeutic agent cyclophosphamide at the start of alemtuzumab treatment would improve its efficacy was tested. Cohorts of secondary recipients were generated and divided into treatment groups as described above. Groups included control mice (treated with PBS injections only); Alemtuzumab only (injected on days 1,4, and 7, as above) treated mice; cyclophosphamide only (cyclophosphamide was dissolved at 15 mg/ml in sterile PBS and delivered via intraperitoneal injections on days 1 and 2; injection volume was weight adjusted, with each mouse receiving 10 uL cyclophosphamide solution per gram of body weight) treated mice; and the combination of Alemtuzumab and cyclophosphamide treated mice. Two days after the completion of alemtuzumab treatments (9 days after the beginning of all treatment), mice were euthanized and the number of GFP+ cells in various organs was determined, as above.

It was found that, although treatment using cyclophosphamide alone reduced the number of GFP+ cells in the peripheral blood as effectively as the treatment using Alemtuzumab alone, Alemtuzumab was ∼15-fold more effective than cyclophosphamide in the liver and the spleen. However, while Alemtuzumab alone had no effect in the brain, cyclophosphamide treatment reduced the number of GFP+ cells found in the brain by ∼300-fold. Nevertheless, neither single treatment had much effect on the leukemia in the bone marrow. The combination of cyclophosphamide and Alemtuzumab was slightly (∼5-10-fold) more effective than Alemtuzumab alone in the blood, liver and spleen, and also slightly (∼5-10-fold) more effective than cyclophosphamide alone in the brain. Notably, however, treating mice with a combination of cyclophosphamide and Alemtuzumab decreased their cancer cell load in the bone marrow by ∼10,000-fold or more, so that few, if any, GFP+ cells were detectable in femoral bone marrow samples after the treatment. See Figures 6A and 6B.

Immunology. 2009 Oct;128(2):260-70. Investigation of the mechanism of action of alemtuzumab in a human CD52 transgenic mouse model. Hu Y, Turner MJ, Shields J, Gale MS, Hutto E, Roberts BL, Siders WM, Kaplan JM.

Leuk Lymphoma. 2008 Dec;49(12):2256-62. Pharmacokinetics of alemtuzumab and the relevance in clinical practice. Elter T, Molnar I, Kuhlmann J, Hallek M, Wendtner C.

Leukemia. 2009 Nov;23(11):1980-8. Epub 2009 Jul 23. Management guidelines for the use of alemtuzumab in chronic lymphocytic leukemia. Osterborg A, Foà R, Bezares RF, Dearden C, Dyer MJ, Geisler C, Lin TS, Montillo M, van Oers MH, Wendtner CM, Rai KR.

Science. 2007 Apr 27;316(5824):600-4. Modeling the initiation and progression of human acute leukemia in mice. Barabé F, Kennedy JA, Hope KJ, Dick JE.

Pediatr Blood Cancer. 2009 Dec;53(6):978-83. A phase II study of Campath-1H in children with relapsed or refractory acute lymphoblastic leukemia: a Children's Oncology Group report. Angiolillo AL, Yu AL, Reaman G, Ingle AM, Secola R, Adamson PC.

Cancer. 2006 Jun 15;106(12):2645-51. Activity of alemtuzumab in patients with CD52-positive acute leukemia. Tibes R, Keating MJ, Ferrajoli A, Wierda W, Ravandi F, Garcia-Manero G, O'Brien S, Cortes J, Verstovsek S, Browning ML, Faderl S.

Cancer. 2010 Mar 1;116(5):1165-76. Adult acute lymphoblastic leukemia: concepts and strategies. Faderl S, O'Brien S, Pui CH, Stock W, Wetzler M, Hoelzer D, Kantarjian HM.

Am J Surg Pathol. 2010 Mar;34(3):327-40. B-cell lymphomas with concurrent IGH-BCL2 and MYC rearrangements are aggressive neoplasms with clinical and pathologic features distinct from Burkitt lymphoma and diffuse large B-cell lymphoma. Snuderl M, Kolman OK, Chen YB, Hsu JJ, Ackerman AM, Dal Cin P, Ferry JA, Harris NL, Hasserjian RP, Zukerberg LR, Abramson JS, Hochberg EP, Lee H, Lee AI, Toomey CE, Sohani AR.

Blood. 2008 Apr 1;111(7):3415-23. Epub 2008 Jan 17. Angiopoietin-like 5 and IGFBP2 stimulate ex vivo expansion of human cord blood hematopoietic stem cells as assayed by NOD/SCID transplantation. Zhang CC, Kaba M, Iizuka S, Huynh H, Lodish HF.

While this invention has been particularly shown and described with references to example embodiments thereof, it will be understood by those skilled in the art that various changes in form and details may be made therein.

## Claims

1. A method of producing a non-human mammal that is a model for a human hematopoietic cancer comprising
a) introducing human hematopoietic stem cells (HSCs) genetically engineered to express the human oncogenes *bcl-2* and *myc* that are associated with human hematopoietic cancer into an immunodeficient non-human mammal; and
b) maintaining the mammal under conditions in which the non-human mammal's blood cell lineage is reconstituted by the human HSCs and the oncogenes are expressed in the mammal,
thereby producing a non-human mammal that is a model for a human hematopoietic cancer.

2. The method of claim 1 wherein the immunodeficient non-human mammal is a mouse; optionally wherein the mouse is selected from the group consisting of: a non-obese diabetic mouse that carries a severe combined immunodeficiency mutation (NOD/scid mouse); a non-obese diabetic mouse that carries a severe combined immunodeficiency mutation and lacks a gene for the cytokine-receptor γ chain (NOD/scid IL2R γ^{-/-} mouse), and a Balb/c rag ^{-/-}γc^{-/-} mouse; optionally wherein (A) the mouse is sublethally irradiated prior to the introduction of the HSCs into the mouse; or (B) wherein the mouse is a model of a human lymphoma or a human leukemia; optionally wherein (a) the lymphoma is follicular lymphoma, Non-Hodgkin's lymphoma, Burkitt's lymphoma, diffuse large B cell lymphoma, and mantle cell lymphoma; and the leukemia is acute lymphoblastic leukemia (ALL), T cell ALL, pro B cell ALL, pre B ALL and naive B ALL, chronic lymphocytic leukemia, acute myeloid leukemia, and chronic myeloid leukemia; or (b) the HSCs are transfected with a virus expressing the *myc* oncogene, the *bcl-2* oncogene or the combination thereof; optionally wherein the virus is a lentivirus; optionally wherein (i) the lentivirus further expresses a gene that encodes a reporter protein; optionally wherein the reporter protein is a green fluorescent protein (GFP), red fluorescent protein (RFP), an antibiotic resistant protein, luciferase, a cell surface protein or a combination thereof; optionally wherein the oncogenes and the reporter gene are operably linked to a cell specific promoter; optionally wherein the cell specific promoter is a human B cell specific promoter; optionally wherein the human B cell specific promoter is a human CD19 promoter; or (ii) the lentivirus is a vesicular stomatitis virus G (VSVG)-pseudotyped lentivirus.

3. The method of claim 1 wherein the human HSCs are CD34+ cells, CD133+ cells, CD34+ CD133+ cells or a combination thereof.

4. The method of claim 1 wherein the HSCs are expanded *in vitro* by culturing the HSCs in serum-free media supplemented with growth factors; optionally wherein (a) the method further comprises introducing a feeder layer of mesenchymal stem cells (MSCs); optionally wherein the growth factors are stem cell factor, thrombopoietin, fibroblast growth factor 1, insulin growth factor binding protein 2 (IGFBP2), angiopoietin-like protein 5 (Angptl5), or a combination thereof; or (b) the HSCs are transfected with the lentivirus during *in vitro* expansion of the HSCs.

5. The method of claim 1 wherein the HSCs are obtained from a cancer patient; optionally wherein the cancer patient has a lymphoid cancer or a leukemia; optionally wherein the lymphoid cancer is follicular lymphoma and the leukemia is acute lymphoblastic leukemia.

6. The method of claim 1 further comprising assessing the reconstitution of the non-human mammal's blood cell lineage by the human HSCs in the non-human mammal; optionally wherein the reconstitution is assessed by detecting human leukocytes that express the human oncogenes *bcl-2* and *myc* in the non-human mammal.

7. The method of claim 1 further comprising producing one or more non-human mammals that are models for a human hematopoietic cancer comprising
c) introducing human cells that express the human oncogenes *bcl-2* and *myc* obtained from the non-human mammal of step b) into the one or more immunodeficient non-human mammals; and
d) maintaining the one or more non-human mammals of c) under conditions in which the one or more non-human mammal's blood cell lineage is reconstituted by the human HSCs and the oncogenes are expressed in the one or more non-human mammals,
thereby producing one or more non-human mammals that are models for a human hematopoietic cancer; optionally wherein (i) the human cells that express the human oncogenes *bcl-2* and *myc* obtained from the non-human mammal of step c) are obtained from the spleen, bone marrow, or a combination thereof of the non-human mammal; optionally wherein the cells are splenocytes; or (ii) the immunodeficient non-human mammal is a mouse; optionally wherein (A) the mouse is selected from the group consisting of: a non-obese diabetic mouse that carries a severe combined immunodeficiency mutation (NOD/scid mouse); a non-obese diabetic mouse that carries a severe combined immunodeficiency mutation and lacks a gene for the cytokine-receptor γ chain (NOD/scid IL2R γ^{-/-} mouse), and a Balb/c rag ^{-/-}γc^{-/-} mouse; optionally wherein the mouse is sublethally irradiated prior to the introduction of the HSCs into the mouse; or (B) the mouse is a model of a human lymphoma or a human leukemia; optionally wherein the lymphoma is follicular lymphoma, Non-Hodgkin's lymphoma, Burkitt's lymphoma, diffuse large B cell lymphoma, and mantle cell lymphoma; and the leukemia is acute lymphoblastic leukemia (ALL), T cell ALL, pro B cell ALL, pre B ALL and naïve B ALL, chronic lymphocytic leukemia, acute myeloid leukemia, and chronic myeloid leukemia.

8. The method of claim 1 further comprising introducing HSCs genetically engineered to comprise nucleic acid that inhibits one or more tumor suppressor genes in the HSCs, in the progeny of the HSCs or in a combination thereof.

9. A non-human mammal that is a model for a human hematopoietic cancer produced by the method of any one of claims 1, 2 or 3; or a non-human mammal that is a model for a human cancer produced by the method of claim 5; or the one or more non-human mammals that are models for a human hematopoietic cancer produced by the method of claim 7.

10. A method of identifying one or more agents or treatment regimens that can be used to treat a human hematopoietic cancer comprising
a) administering the one or more agents to a non-human mammal of claim 9; and
b) determining whether the cancer in the non-human mammal is alleviated, wherein if the cancer in the non-human mammal is alleviated in the non-human mammal, then the one or more agents or treatment protocols can be used to treat the human hematopoietic cancer; optionally (a) wherein the method further comprises comparing whether the cancer in the non-human mammal is alleviated compared to a control; optionally wherein the control is a non-human mammal that has not received the agent or treatment protocols; or (b) wherein two or more agents are administered to the non-human mammal; optionally wherein the administration of the two agents results in a synergistic effect for the treatment of human B cell cancer.

11. A combination consisting of an effective amount of an anti-CD52 antibody and cyclophosphamide for use in treatment of leukemia in an individual.

12. The combination for the use of claim 11, wherein the anti-CD52 antibody is alemtuzumab; optionally wherein the cancer cell load in bone marrow of the individual is reduced 10,000-fold after administration of the alemtuzumab and the cyclophosphamide.

## Patentansprüche

1. Verfahren zur Herstellung eines nicht-menschlichen Säugers, der ein Modell für hämatopoetischen Krebs ist, welches umfasst:
a) Einbringen hämatopoetischer Stammzellen (HSCs), die genetisch verändert wurden, um die humanen, mit hämatopoetischem Krebs assoziierten Onkogene *bcl-2* und *myc* zu exprimieren, in einen immundefizienten, nicht menschlichen Säuger; und
b) Halten des Säugers unter Bedingungen, bei denen die Blutzelllinie des nicht-menschlichen Säugers durch die humanen HSCs wiederhergestellt wird, und die Onkogene exprimiert werden,
wodurch ein nicht-menschlicher Säuger erzeugt wird, der ein Modell für hämatopoetischen Krebs ist.

2. Verfahren nach Anspruch 1, wobei der immundefiziente, nicht-menschliche Säuger eine Maus ist; wahlweise, wobei die Maus ausgewählt ist aus der Gruppe bestehend aus: einer nicht-adipösen, diabetischen Maus, die eine starke kombinierte Immundefizienz-Mutation (NOS/SCID-Maus) trägt; einer nicht-adipösen, diabetischen Maus, die eine starke kombinierte Immundefizienz-Mutation trägt und kein Gen für die Zytokin-Rezeptor-γ-Kette (NOS/SCID-IL2R γ⁻¹-Maus) aufweist, und einer BalB/c rag^{-/-}γc^{-/-} Maus; wahlweise wobei (A) die Maus vor dem Einbringen der HSCs in die Maus sublethal bestrahlt wurde; oder (B) wobei die Maus ein Model eines menschlichen Lymphoms oder menschlicher Leukämie ist; wahlweise wobei (a) das Lymphom follikuläres Lymphom ist, Nicht-Hodgkins Lymphom, Burkitt's Lymphom, diffuses großes B-Zell-Lymphom und Mantelzell-Lymphom ist; und wobei die Leukämie akute lymphoblastische Leukämie (ALL) ist, T-Zell ALL, pro B-Zell ALL, pre B All und naive B ALL, chronische lymphozytische Leukämie, akute myeloide Leukämie und chronische myeloide Leukämie; oder (b) wobei die HSCs transfiziert sind mit einem Virus, der das *myc* Onkogen exprimiert, das *bcl-2* Onkogen oder die Kombination davon; wahlweise wobei das Virus ein Lentivirus ist; wahlweise wobei (i) das Lentivirus weiter ein Gen exprimiert, das ein Reporterprotein kodiert; wobei das Reporterprotein ein grün-fluoreszierendes Protein (GFP) ist, ein rot-fluoreszierendes Protein; ein Antibiotikaresistenz-Protein, Luziferase, ein Zelloberflächenprotein oder eine Kombination davon; wahlweise wobei die Onkogene und das Reportergen betriebsbereit mit einem Zell-spezifischen Promotor verknüpft sind; wahlweise wobei der Zell-spezifische Promotor ein humaner B-Zell spezifischer Promotor ist; wahlweise wobei der humane B-Zell spezifische Promotor ein humaner CD19-Promotor ist; oder (ii) wobei das Lentivirus ein vesikulärer Stomatitis Virus G (VSVG) pseudotypisierter Lentivirus ist.

3. Verfahren nach Anspruch 1, wobei die humanen HSCs CD34⁺-Zellen sind, CD133⁺-Zellen, CD34⁺CD133⁺-Zellen oder eine Kombination davon.

4. Verfahren nach Anspruch 1, wobei die HSCs *in vitro* durch Züchten der HSCs in serumfreien, mit Wachstumsfaktoren komplementierten Medium expandiert werden; wahlweise wobei (a) das Verfahren weiter umfasst, Einbringen einer Nährschicht aus Mesenchymal-Stammzellen (MSCs); wahlweise wobei die Wachstumsfaktoren Stammzell-Faktoren, Thrombopoietin, Fibroblast-Wachstumsfaktor 1, Insulin-Wachstumsfaktor-Bindungsprotein 2 (IGFBP2), Anglopoietin-ähnliches Protein 5 (Angptl5), oder eine Kombination davon, sind; oder (b) wobei die HSCs mit dem Lentivirus während einer *in vitro* Expansion der HSCs transfiziert werden.

5. Verfahren nach Anspruch 1, wobei die HSCs von einem Krebspatient erhalten werden; wahlweise wobei der Krebspatient einen lymphoiden Krebs oder eine Leukämie aufweist; wahlweise wobei der lymphoide Krebs ein follikuläres Lymphom und die Leukämie akute lymphoblastische Leukämie ist.

6. Verfahren nach Anspruch 1, welches weiter umfasst, Bestimmen der Wiederherstellung der Blut-Zellinie des nicht-menschlichen Säugers durch die humanen HSCs in dem nicht-menschlichen Säuger; wahlweise wobei die Wiederherstellung bestimmt wird durch Erfassen humaner Leukozyten, die die humanen Onkogene *bcl-2* und *myc* in dem nicht-menschlichen Säuger exprimieren.

7. Verfahren nach Anspruch 1, welches weiter umfasst, Herstellen ein oder mehrerer nicht-menschlicher Säuger, die Modelle für humanen hämatopoetischen Krebs sind, welches umfasst,
c) Einbringen humaner Zellen, die die humanen Onkogene *bcl-2* und *myc* exprimieren und von dem nicht-menschlichen Säuger von Schritt b) erhalten wurden in den einen oder die mehreren immundefizienten Säuger; und
d) Halten des einen oder der mehreren nicht-menschlichen Säuger von c) unter Bedingungen, bei denen die Blutzelllinie des einen oder der mehreren nicht-menschlichen Säuger durch die humanen HSCs wiederhergestellt wird und die Onkogene in dem einen oder den mehreren nicht-menschlichen Säuger exprimiert werden,
wodurch ein oder mehrere nicht-menschliche Säuger erzeugt werden, die Modelle für humanen hämatopoetischen Krebs sind; wahlweise wobei (i) die von dem nicht-menschlichen Säuger von Schritt c) erhaltenen humanen Zellen, die die humanen Onkogene *bcl-2* und *myc* exprimieren, aus der Milz, dem Knochemark oder einer Kombination davon des nicht-menschlichen Säugers erhalten werden; wahlweise wobei die Zellen Milzzellen sind; oder (ii) wobei der immundefiziente nicht-menschliche Säuger eine Maus ist; wahlweise wobei (A) die Maus ausgewählt ist aus der Gruppe bestehend aus: einer nicht-adipösen diabetischen Maus, die eine starke kombinierte Immundefizienz-Mutation (NOS/SCID-Maus) trägt; einer nicht-adipösen diabetischen Maus, die eine starke kombinierte Immundefizienz-Mutation trägt und kein Gen für die Zytokin-Rezeptor-γ-Kette (NOS/SCID-IL2R γ⁻¹-Maus) aufweist, und einer BalB/c rag^{-/-}γc^{-/-} Maus; wahlweise wobei die Maus vor dem Einbringen der HSCs in die Maus sublethal bestrahlt wurde; oder (B) wobei die Maus ein Model eines menschlichen Lymphoms oder menschlicher Leukämie ist; wahlweise wobei (a) das Lymphom follikuläres Lymphom, Nicht-Hodgkins Lymphom, Burkitt's Lymphom, diffuses großes B-Zell-Lymphom und Mantelzell-Lymphom ist; und wobei die Leukämie akute lymphoblastische Leukämie (ALL) ist, T-Zell ALL, pro B-Zell ALL, pre B All und naive B ALL, chronische lymphozytische Leukämie, akute myeloide Leukämie und chronische myeloide Leukämie.

8. Verfahren nach Anspruch 1, welches weiter umfasst, Einbringen von genetisch veränderten HSCs, welche Nukleinsäuren enthalten, die in den HSCs, in den Nachkommen der HSCs oder in einer Kombination davon ein oder mehrere Tumorsuppressorgene inhibieren.

9. Nicht-menschlicher Säuger, der ein Model für humanen hämatopoetischen Krebs ist, hergestellt nach einem Verfahren nach einem der Ansprüche 1, 2 oder 3; oder ein nicht-menschlicher Säuger, der ein Model für humanen Krebs ist, hergestellt nach dem Verfahren nach Anspruch 5; oder der eine oder die mehreren nicht-menschlichen Säuger, der/die Modelle für humanen hämatopoetischen Krebs ist/sind, hergestellt nach dem Verfahren nach Anspruch 7.

10. Verfahren zur Identifizierung ein oder mehrerer Mittel oder Behandlungs-Regimes, die zur Behandlung eines humanen hämatopoetischen Krebs verwendet können, welches umfasst:
a) Verabreichen des einen oder der mehreren Mittel an einen nicht-menschlichen Säuger nach Anspruch 9; und
b) Bestimmen, ob der Krebs in dem nicht-menschlichen Säuger verringert ist, wobei wenn der Krebs in dem nicht-menschlichen Säuger reduziert ist, anschliessend das eine oder die mehreren Mittel oder Behandlungsprotokolle zur Behandlung des humanen hämatopoetischen Krebs verwendet werden können; wahlweise (a) wobei das Verfahren weiter umfasst, Vergleichen, ob der Krebs in dem nicht-menschlichen Säuger im Vergleich zu einer Kontrolle reduziert ist; wahlweise wobei die Kontrolle ein nicht-menschlicher Säuger ist, der das Mittel oder das Behandlungsprotokolle nicht erhalten hat; oder (b) wobei dem nicht-menschlichen Säuger zwei oder mehrere Mittel verabreicht werden; wahlweise wobei die Verabreichung der zwei Mittel bei der Behandlung von humanem B-Zell Krebs einen synergistischen Effekt aufweist.

11. Kombination, welche aus einer wirksamen Menge eines anti-CD52 Antikörpers und Cyclophosphamid besteht zur Verwendung bei der Behandlung von Leukämie in einem Individuum.

12. Kombination zur Verwendung nach Anspruch 11, worin der anti-CD52 Antikörper Alemtuzumab ist; wahlweise worin die Krebszell-Last im Knochenmark des Individuums nach Verabreichen des Alemtuzumab und des Cyclophosphamids 10.000-fach reduziert ist.

## Revendications

1. Procédé de production d'un mammifère non humain qui est un modèle pour un cancer hématopoïétique humain comprenant
a) l'introduction de cellules souches hématopoïétiques (CSH) humaines génétiquement modifiées pour exprimer les oncogènes humains *bcl-2* et *myc* qui sont associés à un cancer hématopoïétique humain chez un mammifère non humain immunodéficient ; et
b) le maintien du mammifère dans des conditions dans lesquelles la lignée des cellules sanguines du mammifère non humain est reconstituée par les CSH humaines et les oncogènes sont exprimés chez le mammifère,
en produisant de cette façon un mammifère non humain qui est un modèle pour un cancer hématopoïétique humain.

2. Procédé selon la revendication 1, dans lequel le mammifère non humain immunodéficient est une souris ; éventuellement où la souris est choisie dans le groupe constitué de : une souris diabétique non obèse qui porte une mutation d'immunodéficience sévère combinée (souris NOD/scid) ; une souris diabétique non obèse qui porte une mutation d'immunodéficience sévère combinée et à laquelle il manque un gène pour la chaîne γ de récepteur de cytokine (souris NOD/scid IL2R γ^{-/-}), et une souris Balb/c rag^{-/-} γc^{-/-} ; éventuellement où (A) la souris est irradiée à un niveau sublétal avant l'introduction des CSH chez la souris ; ou (B) où la souris est un modèle d'un lymphome humain ou d'une leucémie humaine ; éventuellement où (a) le lymphome est un lymphome folliculaire, un lymphome non hodgkinien, un lymphome de Burkitt, un lymphome diffus à grands lymphocytes B, et un lymphome à cellules du manteau ; et la leucémie est la leucémie lymphoblastique aiguë (LLA), la LLA à lymphocytes T, la LLA à prolymphocytes B, la LLA à prélymphocytes B et la LLA à lymphocytes B naïfs, la leucémie lymphocytaire chronique, la leucémie myéloïde aiguë, et la leucémie myéloïde chronique ; ou (b) les CSH sont transfectées avec un virus exprimant l'oncogène *myc*, l'oncogène *bcl-2* ou leur combinaison ; éventuellement où le virus est un lentivirus ; éventuellement où (i) le lentivirus exprime en outre un gène qui code pour une protéine rapporteur ; éventuellement où la protéine rapporteur est une protéine fluorescente verte (GFP), une protéine fluorescente rouge (RFP), une protéine résistante à un antibiotique, la luciférase, une protéine de la surface cellulaire ou l'une de leurs combinaisons ; éventuellement où les oncogènes et le gène rapporteur sont liés de façon fonctionnelle à un promoteur spécifique de la cellule ; éventuellement où le promoteur spécifique de la cellule est un promoteur spécifique des lymphocytes B humains ; éventuellement où le promoteur spécifique des lymphocytes B humains est un promoteur du CD19 humain ; ou (ii) le lentivirus est un lentivirus pseudotypé par le virus de la stomatite vésiculaire G (VSVG).

3. Procédé selon la revendication 1, dans lequel les CSH humaines sont des cellules CD34+, des cellules CD133+, des cellules CD34+ CD133+ ou l'une de leurs combinaisons.

4. Procédé selon la revendication 1, dans lequel les CSH sont expansées *in vitro* par la culture des CSH dans du milieu dépourvu de sérum complémenté avec des facteurs de croissance ; éventuellement où (a) le procédé comprend en outre l'introduction d'une couche nourricière de cellules souches mésenchymateuses (CSM) ; éventuellement où les facteurs de croissance sont le facteur des cellules souches, la thrombopoïétine, le facteur de croissance des fibroblastes 1, la protéine 2 de liaison du facteur de croissance insulinique (IGFBP2), la protéine de type angiopoïétine 5 (Angptl5), ou l'une de leurs combinaisons ; ou (b) les CSH sont transfectées avec le lentivirus durant l'expansion *in vitro* des CSH.

5. Procédé selon la revendication 1, dans lequel les CSH sont obtenues d'un patient cancéreux ; éventuellement où le patient cancéreux souffre d'un cancer lymphoïde ou d'une leucémie ; éventuellement où le cancer lymphoïde est un lymphome folliculaire et la leucémie est une leucémie lymphoblastique aiguë.

6. Procédé selon la revendication 1 comprenant en outre l'estimation de la reconstitution de la lignée des cellules sanguines du mammifère non humain par les CSH humaines chez le mammifère non humain ; éventuellement où la reconstitution est estimée par la détection des leucocytes humains qui expriment les oncogènes humains *bcl-2* et *myc* chez le mammifère non humain.

7. Procédé selon la revendication 1 comprenant en outre la production d'un ou de plusieurs mammifères non humains qui sont des modèles pour un cancer hématopoïétique humain comprenant
c) l'introduction de cellules humaines qui expriment les oncogènes humains *bcl-2* et *myc* obtenus du mammifère non humain de l'étape b) chez les un ou plusieurs mammifères non humains immunodéficients ; et
d) le maintien des un ou plusieurs mammifères non humains de c) dans des conditions dans lesquelles la lignée des cellules sanguines des un ou plusieurs mammifères non humains est reconstituée par les CSH humaines et les oncogènes sont exprimés chez les un ou plusieurs mammifères non humains,
en produisant de cette façon un ou plusieurs mammifères non humains qui sont des modèles pour un cancer hématopoïétique humain ; éventuellement où (i) les cellules humaines qui expriment les oncogènes humains *bcl-2* et *myc* obtenues du mammifère non humain de l'étape c) sont obtenues à partir de la rate, la moelle osseuse, ou l'une de leurs combinaisons du mammifère non humain ; éventuellement où les cellules sont des splénocytes ; ou (ii) le mammifère non humain immunodéficient est une souris ; éventuellement où (A) la souris est choisie dans le groupe constitué de : une souris diabétique non obèse qui porte une mutation d'immunodéficience sévère combinée (souris NOD/scid) ; une souris diabétique non obèse qui porte une mutation d'immunodéficience sévère combinée et à laquelle il manque un gène pour la chaîne γ de récepteur de cytokine (souris NOD/scid IL2R γ^{-/-}), et une souris Balb/c rag^{-/-} γc^{-/-} ; éventuellement où la souris est irradiée à un niveau sublétal avant l'introduction des CSH chez la souris ; ou (B) la souris est un modèle d'un lymphome humain ou d'une leucémie humaine ; éventuellement où le lymphome est un lymphome folliculaire, un lymphome non hodgkinien, un lymphome de Burkitt, un lymphome diffus à grands lymphocytes B, et un lymphome à cellules du manteau ; et la leucémie est la leucémie lymphoblastique aiguë (LLA), la LLA à lymphocytes T, la LLA à prolymphocytes B, la LLA à prélymphocytes B et la LLA à lymphocytes B naïfs, la leucémie lymphocytaire chronique, la leucémie myéloïde aiguë, et la leucémie myéloïde chronique.

8. Procédé selon la revendication 1 comprenant en outre l'introduction de CSH génétiquement modifiées pour comprendre un acide nucléique qui inhibe un ou plusieurs gènes suppresseurs de tumeurs dans les CSH, dans la descendance des CSH ou dans l'une de leurs combinaisons.

9. Mammifère non humain qui est un modèle pour un cancer hématopoïétique humain produit par le procédé selon l'une quelconque des revendications 1, 2 ou 3 ; ou mammifère non humain qui est un modèle pour un cancer humain produit par le procédé selon la revendication 5 ; ou un ou plusieurs mammifères non humains qui sont des modèles pour un cancer hématopoïétique humain produits par le procédé selon la revendication 7.

10. Procédé d'identification d'un ou de plusieurs agents ou régimes thérapeutiques qui peuvent être utilisés pour traiter un cancer hématopoïétique humain comprenant
a) l'administration des un ou plusieurs agents à un mammifère non humain selon la revendication 9 ; et
b) la détermination si le cancer chez le mammifère non humain est soulagé, où si le cancer chez le mammifère non humain est soulagé chez le mammifère non humain, alors les un ou plusieurs agents ou protocoles de traitement peuvent être utilisés pour traiter le cancer hématopoïétique humain ; éventuellement (a) où le procédé comprend en outre la comparaison si le cancer chez le mammifère non humain est soulagé comparativement à un témoin ; éventuellement où le témoin est un mammifère non humain qui n'a pas reçu l'agent ou les protocoles de traitement ; ou (b) où deux agents ou plus sont administrés au mammifère non humain ; éventuellement où l'administration des deux agents entraîne un effet synergique pour le traitement du cancer à lymphocytes B humain.

11. Combinaison constituée d'une quantité efficace d'un anticorps anti-CD52 et de cyclophosphamide pour une utilisation dans le traitement de la leucémie chez un individu.

12. Combinaison pour l'utilisation selon la revendication 11, où l'anticorps anti-CD52 est l'alemtuzumab ; éventuellement où la charge des cellules cancéreuses dans la moelle osseuse de l'individu est réduite de 10 000 fois après l'administration de l'alemtuzumab et du cyclophosphamide.
